# EUROPÄISCHE PATENTSCHRIFT

(11) **EP 1 181 898 B1**
(45) Veröffentlichungstag und Bekanntmachung des Hinweises auf die Patenterteilung: **19.10.2005**
(21) Anmeldenummer: 01118891.9
(22) Anmeldetag: 17.08.2001
(51) Int. Cl.: A61C 13/30

(54) **Formkappe für dentale Stiftaufbauten**
Cap for upper structure of dental post
Manchon pour superstructure de tenon dentaire

(30) Priorität: 21.08.2000 DE 10040772; 07.12.2000 DE 10060922
(43) Veröffentlichungstag der Anmeldung: 27.02.2002
(73) Patentinhaber: Ivoclar Vivadent AG, 9494 Schaan (LI)
(72) Erfinder: Hagenbuch, Konrad, 9472 Grabs (CH); Zanghellini, Gerhard, 9494 Schaan (LI)
(74) Vertreter: UEXKÜLL & STOLBERG

(56) Entgegenhaltungen:
- DE-A- 4 334 608
- DE-U- 8 714 974
- US-A- 2 237 350
- US-A- 5 328 372

## Beschreibung

Die Erfindung betrifft Formkappen für dentale Stiftaufbauten sowie Kits und Verfahren zur Herstellung dentaler Stiftaufbauten.

Zur Befestigung von Zahnrestaurationen und zum Wiederaufbau fehlender Zahnhartsubstanz werden Verankerungsstifte verwendet. Diese werden meist in den Wurzelkanal eingesetzt und daher auch als Wurzelstifte bezeichnet. Neben der intrakanalären Verankerung ist jedoch auch eine parapulpäre Verankerung üblich.

Das Einsetzen dieser Stifte in den Wurzelkanal erfolgt in drei Phasen. In der ersten Phase wird der Wurzelkanal mit üblichen Wurzelkanalinstrumenten bis zum Apex aufbereitet. In der zweiten Phase wird der koronale Wurzelanteil mit genormten Bohrern bearbeitet. In der dritten Phase wird der Verankerungs- oder Wurzelstift in den aufbereiteten Kanal eingesetzt.

Anschließend wird ein sogenannter Stift- oder Stumpfaufbau an den Verankerungsstift modelliert. Die Aufbauten können mit plastischen Materialien wie Amalgam und Komposit direkt im Mund des Patienten aufgebaut und formgerecht beschliffen werden. Sie können aber auch indirekt nach Abformen der präparierten Zahnhartsubstanz mit dem gesetzten Stift im zahntechnischen Labor hergestellt werden. Dieser Aufbau wird schließlich überkront oder verblendet.

Zur Herstellung von Stiftaufbauten aus Kompositmaterialien können als Hilfsmittel Kappen aus flexiblem Kunststoff, wie Silicon oder Polyethylen, eingesetzt werden, welche die leichte Formung eines Standardstumpfes ermöglichen. Die Kappe wird gewöhnlich mit Komposit gefüllt, über den Wurzelstift gestülpt und das Kompositmaterial anschließend gehärtet. Die Kappen bestehen aus weichem, flexiblem Material und lassen sich nach dem Härten leicht von dem gehärtetem Kompositmaterial entfernen. Danach wird der Stumpfaufbau weiter bearbeitet. Die Kappen sind für Front- und Backenzähne in unterschiedlichen Größen erhältlich. Die zur Herstellung der Aufbauten eingesetzten Komposite enthalten neben einem polymerisierbaren Matrixmaterial üblicherweise partikuläre Füllstoffe. Die Belastbarkeit der Aufbauten ist begrenzt.

Die DE 43 34 608 A1 offenbart ein System zur Restauration zerstörter Zähne, das einen Wurzelstift mit einem einstückig ausgebildetem Retentionskopf, eine am Retentionskopf verankerbare, vorkonfektionierte Kappen und eine auf die Kappe aufbringbare Verblendkrone enthält. Die Kappe ist bodenseitig mit einer Montageausnehmung versehen, in welche der Retentionskopf einbringbar ist, und wird mittels einer Fixiermasse mit diesem verbunden. Die Kappen sind vorzugsweise aus, keramischen Materialien gefertigt, die sich dadurch auszeichnen, daß sie im Vergleich zu Kunststoffkappen weit weniger elastisch sind.

Die US 5,328,372 offenbart ein Krone-Wurzel-Wiederherstellungssystem, das einen Wurzelstift aus faserverstärktem Kunststoff und ein Material zur Wiederherstellung der Krone enthält, das zwischen Wurzelstift und Dentin gebracht wird. Das Material zur Wiederherstellung der Krone ist ein selbsthärtendes Polyurethan-Acrylharz, das mit Glasfasern gefüllt ist.

Aufgabe der Erfindung ist die Bereitstellung von Formkappen zur Herstellung dentaler Stiftaufbauten mit erhöhter Festigkeit.

Diese Aufgabe wird durch Formkappen gelöst, die als fester Bestandteil des Stiftaufbaus geeignet sind.

Die Formkappen bestehen aus Kunststoff, der neben einem organischen Matrixmaterial faserförmigen Füllstoff enthält und weisen einen E-Modul von mindestens 2000 MPa auf.

Weiter bevorzugt sind zahnfarbene und insbesondere lichtdurchlässige Formkappen, so daß das darin enthaltene polymerisierbare Material durch Licht gehärtet werden kann.

Als Matrixmaterial eignen sich besonders ionisch und/oder radikalisch polymerisierbare mono- oder multifunktionelle Monomere, insbesondere Mono(meth)acrylate, wie Methyl-, Ethyl-, Butyl-, Benzyl-, Furfuryl- oder Phenyl(meth)acrylat, mehrfunktionelle Acrylate und Methacrylate wie zum Beispiel Bisphenol-(A)-di(meth)acrylat, Decandioldi(meth)-acrylat, Butandiol-di(meth)acrylat, 1,10-Decandioldi(meth)acrylat und/oder 1,12-Dodecandioldi(meth)acrylat.

Besonders bevorzugte Matrixmaterialien sind Polycarbonat-di(meth)acrylate, insbesondere das Kondensationsprodukt aus einem Hydroxyalkylmethacrylat, vorzugsweise 2-Hydroxyethylmethacrylat, und einem Bis(chlorformiat), vorzugsweise Triethylenglykolbis(chlorformiat)). Polycarbonat-Tri- oder Tetra-(meth)-acrylate, Urethandi-, tri-, tetra-(meth)acrylate und Mischungen davon. Monomere dieses Typs werden in der DE 36 32 868 A1 und der US 5,444,104 beschrieben.

Weitere besonders bevorzugte Monomere sind Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- (TEGDMA) und Tetraethylenglykoldi(meth)acrylat, Trimethylolpropantri(meth)-acrylat und Pentaerythrittetra(meth)acrylat.

Zur Initiierung der radikalischen Polymerisation enthält die polymerisierbare Komponente thermische und/oder vorzugsweise Photoinitiatoren.

Bevorzugte Initiatoren für die thermische Härtung sind Peroxide, wie beispielsweise Dibenzoylperoxid, Dilaurylperoxid, tert.-Butylperoctoat und tert.-Butylperbenzoat sowie Azobisisobutyroethylester, Benzpinakol und 2,2-Dimethylbenzpinakol.

Bevorzugte Photoinitiatoren sind Benzophenon und Benzoin sowie deren Derivate, α-Diketone und deren Derivate, wie beispielsweise 9,10-Phenanthrenchinon, Diacetyl und 4,4-Dichlorbenzil. Besonders bevorzugte Photoinitiatoren sind Champherchinon und 2,2-Methoxy-2-phenyl-acetophenon und insbesondere Kombinationen von α-Diketonen mit Aminen als Reduktionsmittel, wie zum Beispiel N-Cyanoethyl-N-methylanilin, 4-(N,N-Dimethylamino)-benzoesäureester,N,N-Dimethylaminoethylmethacrylat,N,N-Dimethyl-sym.-xylidin oder Triethanolamin. Darüber hinaus sind Acylphosphine, wie zum Beispiel 2,4,6-Trimethylbenzoyldiphenyl- oder Bis-(2,6-dichlorbenzoyl)-4-N-propylphenylphosphinoxid, als Photoinitiatoren geeignet.

Für die duale Aushärtung von radikalisch und kationisch polymerisierbaren Systemen eignen sich besonders Diaryliodonium- oder Triarylsulfoniumsalze, wie zum Beispiel Triphenylsulfoniumhexafluorophosphat und -hexafluoroantimonat.

Als Initiatoren für eine Polymerisation bei Raumtemperatur werden Redox-Initiatorkombinationen, wie zum Beispiel Kombinationen von Benzoyl- oder Laurylperoxid mit N,N-Dimethyl-sym.-xylidin oder N,N-Dimethyl-p-toluidin, verwendet. Weitere geeignete Initiatoren und Beschleuniger sind Vitamin C und Barbitursäure.

Das Matrixmaterial enthält neben polymerisierbaren Monomeren und/oder Präpolymeren vorzugsweise auch Füllstoffe. Als Füllstoffe sind organische und anorganische faserförmige Materialien, wie Fasern, Fasermatten und/oder Gewebe bevorzugt. Bevorzugt sind Glasfasern, Polyethylenfasern (Spectra, Dynema), Polyamid-, insbesondere Aramidfasern (Kevlar), und Carbonfasern sowie Matten und Gewebe dieser Fasern.

Die faserförmigen Materialien weisen vorzugsweise einen Faserdurchmesser von < 0,25 mm, insbesondere 0,01 bis 0,25 mm, und ein Verhältnis von Faserlänge zu Faserdurchmesser von > 10 : 1, insbesondere > 10 : 1 bis 100 : 1 auf.

Als faserförmige Füllstoffe sind weiterhin auch sogenannte Whiskers bevorzugt. Hierbei handelt es sich um Mikrofasern mit einer Länge von vorzugsweise 10 bis 200 µm und einem Durchmesser von vorzugsweise 0,1 bis 1 µm.

Alternativ oder zusätzlich zu den faserförmigen Füllstoffen kann das Kappenmaterial partikuläre Füllstoffe enthalten. Bevorzugte partikuläre Füllstoffe sind gefällte oder gemahlene Kunststoffpartikel, vorzugsweise mit einer Korngröße von 0,02 bis 100 µm; Hybrid-Füllstoffe, wie gemahlenes Polymerisat aus einer organischen Matrix mit organischen und/oder anorganischen Füllstoffen, wobei das gemahlene Polymerisat vorzugsweise eine Korngröße von 0,5 bis 80 µm aufweist; und/oder anorganische Füllstoffe.

Besonders bevorzugte partikuläre Füllstoffe sind amorphe kugelförmige Materialien auf der Basis von Mischoxiden aus SiO₂, ZrO₂ und/oder TiO₂ (DE 40 29 230 A1), mikrofeine Füllstoffe, wie pyrogene Kieselsäure oder Fällungskieselsäure, sphärische SiO₂-Partikel (gefällte Partikel) mit einer Korngröße von 200 bis 700 nm sowie Makro- (Partikelgröße von 5 µm bis 200 µm) oder Minifüllstoffe (Partikelgröße von 0,5 bis 5 µm), wie Quarz-, Glaskeramik- oder Glaspulver mit einer durchschnittlichen Teilchengröße von 0,5 µm bis 5 µm sowie röntgenopake Füllstoffe, wie Ytterbiumtrifluorid.

Die organischen und insbesondere die anorganischen Füllstoffe sind vorzugsweise mit einem geeigneten Haftvermittler versehen, d.h. beispielsweise silanisiert, um eine feste Bindung zwischen Faser und Matrix zu gewährleisten. Geeignete Silane sind dem Fachmann bekannt. Bevorzugte Silane sind gamma-Methacryloxypropyl-trimethoxy-silan (A-174) und gamma-Methacryloxypropyltris(2-methoxyethoxy)-silan (A-175). Diese Silane eignen sich insbesondere dann, wenn das Matrixmaterial polymerisierbare (Meth)acrylatgruppen aufweist.

Die Mischungen können darüber hinaus weitere Additive wie Färbemittel (Pigmente und Farbstoffe), Stabilisatoren, Aromastoffe, mikrobiozide Wirkstoffe, Weichmacher und/oder UV-Absorber enthalten.

Besonders bevorzugte Kappenmaterialien sind faserverstärkte Kunststoffe auf der Basis von Urethandimethacrylat, die zusätzlich mit partikulärem anorganischem Füllstoff, vorzugsweise Glaspulver verstärkt sind, wie beispielsweise Materialien folgender Zusammensetzung:

| Komponente | Anteil (Gew.-%) |
|---|---|
| Urethandimethacrylat | 10 bis 20 % |
| Glasfasern (silanisiert) | 60 bis 70 % |
| Glaspulver (1 µm, silanisiert) | 15 bis 20 % |
| pyrogene Kieselsäure (Aerosil) | 0,5 bis 5 % |
| Katalysator | 0,02 bis 0,5 % |

Weitere bevorzugte Kappenmaterialien sind mit organischem Bindemittel imprägnierte Gewebe wie beispielsweise:

| Komponente | Anteil (Gew.-%) |
|---|---|
| Bis-GMA | 33 bis 43 %, z.B. 38 % |
| Triethylenglykoldimethacrylat | 5 bis 15 %, z.B. 10 % |
| hochdisperses SiO₂ | 3 bis 10 %, z.B. 6 % |
| Katalysatoren und Stabilisatoren | 0,5 bis 2 %, z.B. 0,5 % |
| Glasfasern (Gewebe, achtlagig) | 40 bis 50 %, z.B. 45,5 % |

Als Kappenmaterialien sind weiterhin thermoplastische Kunststoffe bevorzugt, insbesondere solche mit einer Glasumwandlungstemperatur (Tg) von mehr als 40 °C, vorzugsweise mehr als 80 °C und insbesondere mehr als 100 °C. Besonders bevorzugt sind Kunststoffe mit einem Elastizitätsmodul (E-Modul, bestimmt gemäß EN ISO 178) von mehr als 2000 MPa (gemessen bei Raumtemperatur), insbesondere mehr als 5000 MPa. Zu den besonders bevorzugten thermoplastischen Kunststoffen zählen Polymethylmethacrylat (PMMA, Tg = 105 °C, E-Modul = 3300 MPa), Polysulfon (Tg = 190 °C, E-Modul = 2700 MPa) und Polycarbonat (Tg = 145 °C, E-Modul = 2300 MPa). Eine Erhöhung des E-Moduls der Kunststoffe läßt sich durch den Zusatz von Füllstoffen, insbesondere faserförmigen Füllstoffen erreichen. Beispielsweise weist Polysulfon, das mit 25 Gew.-% Glasfasern gefüllt ist, einen E-Modul von 7200 MPa auf. Somit sind füllstoffhaltige thermoplastische Kunststoffe bevorzugt, insbesondere faserförmigen Füllstoff enthaltende thermoplastische Kunststoffe. Der E-Modul kann Werte von 25.000 MPa oder gar 50.000 MPa annehmen.

Die Matrix der Formkappen kann in ungehärteter, vorgehärteter oder vollständig ausgehärteter Form vorliegen. Wesentlich ist, das die Kappen ihre Form beibehalten. Die Verwendung eines vorgehärteten, d.h. teilweise polymerisierten Materials ist bevorzugt.

Die Oberfläche, insbesondere die innere Oberfläche der Formkappen ist mit Mitteln ausgestattet, die einen festen Verbund zwischen Kappe und Kappenfüllmaterial gewährleisten. Kappen aus ungehärtetem oder teilweise gehärtetem Material enthalten polymerisierbare Gruppen, die bei der späteren Härtung durch chemische Bindungen eine feste Haftung zwischen Kappe und Kappenfüllmaterial sicherstellen. Bei der Verwendung eines vollständig gehärteten Materials sowie bei Metall- und Keramikkappen wird die Oberfläche der Kappen vorzugsweise so modifiziert, daß diese polymerisierbare Gruppen aufweist. Polymerisierbare Gruppen lassen sich nachträglich beispielsweise durch Silanisieren der Kappen auf die Kappenoberfläche aufbringen. Im Fall von Kappen aus Kunststoff ist es vorteilhaft, wenn der Kunststoff anorganischen Füllstoff, beispielsweise Glaspulver oder Glasfasern, enthält, da Silanisierungsmittel bevorzugt mit an der Kappenoberfläche befindlichen Füllstoffpartikeln oder Füllstoffasern reagieren. Als Silanisierungsmittel sind die oben genannten Silane bevorzugt.

Bevorzugte polymerisierbare Gruppen sind radikalisch polymerisierbare Gruppen, insbesondere ethylenisch ungesättigte Gruppen wie Vinyl-, Allyl-, Acryl- und Methacrylgruppen.

Zur Verbesserung der Haftung zwischen Kappe und Kappenfüllmaterial können die Kappen auch mit einem Lösungsmittel oder einem reaktiven Verdünner behandelt werden, so daß das Kappenmaterial oberflächlich anquillt. Diese Variante eignet sich besonders für vollständig gehärtete Kunststoffkappen.

Darüber hinaus kann eine feste Haftung durch mechanische Mittel bewirkt werden. Beispielsweise kann die Kappenoberfläche zur Haftungsverbesserung durch Sandstrahlen aufgerauht werden oder die Oberfläche kann mit Retentionen, wie Unterschnitten, Rillen oder Perforationen, versehen werden.

Keramikkappen oder Kappen aus Kunststoffen, die anorganische Füllstoffe enthalten, werden zur Gewährleistung einer festen Bindung zwischen Kappe und Kappenfüllmaterial vorzugsweise angerauht und silanisiert.

Metallische Kappen werden zur Verbesserung der Haftung vorzugsweise mit (Meth)acrylatgruppen enthaltenden Phosphorsäureestern, wie z.B. dem Produkt Targis Link der Firma Ivoclar, behandelt. Die Phosphorsäuregruppen dieser Ester reagieren mit der Metalloberfläche oder oberflächlich vorhandenen Metalloxiden unter Ausbildung von Phosphatbindungen, die (Meth)acrylatgruppen sind polymerisierbar und können mit dem Kappenfüllmaterial reagieren. Weiterhin kann auf die Metalloberfläche eine dünne glasartige Schicht, eine sogenannte SiOₓ-C-Schicht, aufgebracht werden, die sich z.B. mit den oben genannten Silanen silanisieren läßt. Hierzu wird die Metalloberfläche beispielsweise mit dem Produkt Silicoater® der Firma Kulzer behandelt. Zudem können durch Sandstrahlen mit einem speziellen Strahlmittel (Rocatec®, Firma ESPE) silikatische Teilchen auf der Metalloberflche verankert werden, die eine dünne keramische Schicht bilden, welche sich ebenfalls silanisieren läßt.

Zur Herstellung von Stift- oder Stumpfaufbauten werden die Kappen mit einem polymerisierbaren Material, vorzugsweise einem polymerisierbaren Kompositmaterial gefüllt (Kappenfüllmaterial) und auf den präparierten Zahn oder ein Modell davon aufgesetzt. Vor dem Füllen kann die Kappe gegebenenfalls mit einer Schere zugeschnitten werden. Danach wird das polymerisierbare Material und ggf. die Kappe gehärtet, vorzugsweise durch Photopolymerisation. Hierbei wird das Material fest mit der Kappe verbunden.

Als Kappenfüllmaterial eignen sich besonders Mischungen der oben genannten Monomere, Polymerisationsinitiatoren und vorzugsweise auch Füllstoffe, wobei als Füllstoffe nicht-faserförmige Füllstoffe bevorzugt sind. Bevorzugt sind Materialien, die 20 bis 80 Gew.-% eines oder mehrerer polymerisierbarer Monomere, 20 bis 80 Gew.-% Füllstoff und 0,05 bis 2 Gew.-% Polymerisationsinitiator enthalten. Das Kappenfüllmaterial enthält vorzugsweise einen Initiator für die Photopolymerisation und ist durch Licht härtbar. Besonders bevorzugte Kappenfüllmaterialien haben die folgende Zusammensetzungen:

| Feinpartikelhybrid | |
|---|---|
| Komponente | Anteil (Gew.-%) |
| Bis-GMA | 6 bis 12 %, z.B. 8,7 % |
| Decandioldimethacrylat | 3 bis 7 %, z.B. 4,7 % |
| Urethandimethacrylat | 6 bis 14 %, z.B. 9,0 % |
| Bariumglasfüller (silanisiert) | 60 bis 85 %, z.B. 72,0 % |
| hochdisperses SiO₂ | 3 bis 7 %, z.B. 5,0 % |
| Katalysatoren und Stabilisatoren | 0,2 bis 1 %, z.B. 0,6 % |

| Mikrogefülltes Composite | |
|---|---|
| Komponente | Anteil (Gew.-%) |
| ethoxyliertes Bisphenol-A-dimethacrylat | 36,0 bis 68,0 %, z.B. 53,2 % |
| Triethylenglycoldimethacrylat | 11,0 bis 17,0 %, z.B. 13,3 % |
| Antioxidationsmittel | 0,002 bis 0,02% |
| z.B. butyliertes Hydroxytoluol | 0,008% |
| Härter | 0,06 bis 0,20 % |
| z.B. 2,3-Bornandion | 0,12 % |
| Härtungsbeschleuniger | 0,05 bis 0,20 % |
| z.B. Ethyl-4-dimethylaminobenzoat | 0,12% |
| submicrones silanisiertes SiO₂ | 15,0 bis 55,0 %, z.B. 33,3 % |
| als submicrones SiO₂ wird vorzugsweise Aerosil mit einer Partikelgröße von 0,01 bis 0,04 µm und z.B. einer durchschnittlichen Partikelgröße von 0,02 µm verwendet | |

| Makrogefülltes Composite | |
|---|---|
| Komponente | Anteil (Gew.-%) |
| Bindemittel | |
| Bis-GMA | 55 bis 66 %, z.B. 61,2 % |
| Bisphenol-A-dimethacrylat | 5 bis 9 %, z.B. 6,8 % |
| TEGDMA | 20 bis 34 %, z.B. 26,9 % |
| Methacrylsäure | 1,5 bis 2,5 %, z.B. 2,0 % |
| Benzil | 0,1 bis 0,5 %, z.B. 0,3 % |
| Campherchinon | 0,1 bis 0,5 %, z.B. 0,3 % |
| 2-(N,N-Dimethylamino)ethyl- | |
| methacrylat | 2 bis 3 %, z.B. 2,5 % |
| Die Prozentangaben beziehen sich auf die Gesamtmasse des Bindemittels. | |
| 20 bis 35 Gew.-% des Bindemittels werden mit 65 bis 80 Gew.-% silanisiertes Ba-Al-Silikatglas als Füllstoff zu dem fertigen Composite gemischt, z.B. 28 Gew.-% des Bindemittels und 72 Gew.-% des Füllstoffs. | |

Die Formkappen werden bei der Polymerisation fest mit dem polymerisierbaren Material verbunden und bewirken so eine deutliche Erhöhung der Festigkeit des Wurzelstiftaufbaus ohne zusätzliche Arbeitsschritte erforderlich zu machen. Vielmehr entfällt das Entfernen der bisher zum Formen der Stiftaufbauten verwendeten Formkappen. Die Kappe dient als Grundform des Stumpfes. Dieser kann später beispielsweise verblendet oder überkront werden.

Die Formkappen bilden im Gegensatz zu bisherigen Formkappen einen integralen Bestandteil der Dentalrestauration, d.h. die Kappen verbleiben permanent in der Restauration und übernehmen dort eine tragende Funktion. Die bisher zur Herstellung von Stiftaufbauten verwendeten Formkappen aus weichem, flexiblem Kunststoff sind für diesen Zweck ungeeignet da sie eine ungenügende mechanischen Festigkeit aufweisen. Diese Kappen sind zudem so gestaltet, daß sie sich nach der Härtung des Kappenfüllmaterials wieder gut entfernen lassen. Die erfindungsgemäßen Formkappen weisen im ausgehärtetem Zustand einen E-Modul von mindestens 2000 MPa, besonders bevorzugt von 5000 MPa auf. Ganz besonders bevorzugt sind Kappen mit einem E-Modul von 10.000 MPa bis 50.000 MPa und insbesondere von etwa 30.000 MPa (bestimmt gemäß EN ISO 178 bei Raumtemperatur, ohne Kappenfüllmaterial).

Die Formkappen sind in Form und Größe an die Verwendung im Dentalbereich angepaßt. Bevorzugt sind Kappen, die auf die Form und Größe des zu behandelnden Zahns, wie Schneidezahn, Eckzahn, Prämolar oder Molar, abgestimmt sind. Die Kappen haben an der Basis vorzugsweise einen ovalen Querschnitt mit einem Durchmesser in Längsrichtung von 6 bis 11 mm und einen Durchmesser in transversaler Richtung von 4 bis 9 mm. Die Kappen können beispielsweise einen elliptischen Querschnitt mit einem Verhältnis von großer zu kleiner Halbachse von 1,22 bis 1,68 aufweisen. Die Höhe der Kappen beträgt vorzugsweise 6 bis 10 mm.

Die erfindungsgemäßen Formkappen bewirken eine deutliche Erhöhung der Festigkeit des gesamten Wurzelstiftaufbaus und bewahren diesen vor übermäßigen Spannungsspitzen. Die Herstellung von Stiftaufbauten wird zudem deutlich vereinfacht, da einerseits das Entfernen der Formkappe entfällt, andererseits die Verwendung genormter Formkappen die Anpassung z.B. der Krone erleichtert.

Formkappe und polymerisierbares Material können getrennt oder vorzugsweise gemeinsam als Kit vertrieben werden. Gemäß einer besonders bevorzugten Ausführungsform werden mit polymerisierbarem Material gefüllte Formkappen vertrieben. Hierdurch entfällt das bisher erforderliche Füllen durch den Zahnarzt oder den Zahntechniker. Kits und Formkappen, die polymerisierbare Materialien enthalten, werden vorzugsweise in sauerstoff- und ggf. lichtundurchlässigen Verpackungen vertrieben, um ein vorzeitiges Härten der Materialien zu verhindern.

Weiterer Gegenstand der Erfindung sind Kits zur Herstellung von Zahnstiftaufbauten, die mindestens eine der oben beschriebenen Formkappen, mindestens einen Verankerungsstift und polymerisierbares Material enthalten. Die Verankerungsstifte können aus Metall, Keramik oder vorzugsweise faserverstärktem Kunststoff hergestellt sein. Die Kits können darüber hinaus ein Material zur Verblendung der Formkappen enthalten. Die Kits enthalten vorzugsweise eine Formkappe auf der Basis von faserverstärktem Kunststoff und eignen sich somit insbesondere zur Herstellung faserverstärkter Stiftaufbauten.

Alternativ können die Kits Kronen oder Kronenrohlinge enthalten, die vorzugsweise bereits auf die Formkappengröße abgestimmt sind.

Zum Herstellen einer Zahnrestauration wie beispielsweise einer Krone wird der zu behandelnde Zahn zunächst vom Zahnarzt beschliffen und anschließend auf die oben beschriebene Weise mit einem intrakanalären oder parapulpären Verankerungsstift versehen. Dann wird eine passende Formkappe ausgewählt, die Kappe falls erforderlich mit einem polymerisierbaren Material gefüllt, die gefüllte Formkappe auf den Verankerungsstift aufgesetzt und das polymerisierbare Material gehärtet. Hierbei wird dieses fest mit der Formkappe und dem Verankerungsstift verbunden. Das Konstrukt aus Verankerungsstift, Kappenfüllmaterial und Formkappe wird als Stift- oder Stumpfaufbau bezeichnet.

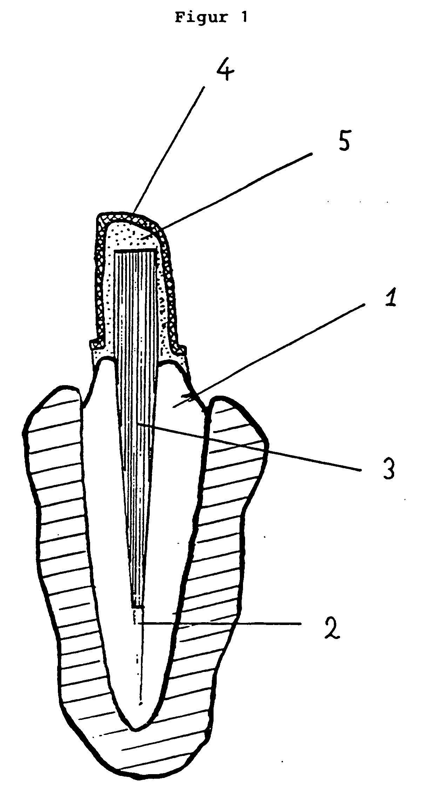

Figur 1 zeigt eine Zahnwurzel 1 mit einem Wurzelkanal 2. In den Wurzelkanal 2 ist ein Wurzelstift 3 eingesetzt. Über den Wurzelstift 3 ist eine faserverstärkte Formkappe 4 gestülpt, die mit polymerisierbarem Material 5 gefüllt ist.

Danach wird entweder ein Abdruck des so präparierten Zahns genommen und ein Positivmodell für die Anfertigung der Krone in einem Dentallabor angefertigt oder der Zahn direkt im Munde des Patienten weiter versorgt. Im ersten Fall wird die fertiggestellte Krone mit einem Befestigungskomposit an dem präparierten Zahn befestigt. Im zweiten Fall wird entweder eine an die Formkappe angepaßte Fertigkrone auf den präparierten Zahn aufgesetzt und beschliffen oder es wird durch Aufbringen von Verblendmaterial auf den präparierten Zahn eine Krone geformt. Fertigkronen eignen sich besonders zur temporären Versorgung des Zahns bis zur Fertigstellung der endgültigen Krone.

Zwei Formkappen, die auf die eine Zahnlücke begrenzenden Zähnen aufgesetzt werden, können als Basis für eine Brücke dienen.

Die Herstellung der Dentalrestauration kann auch in einem zahntechnischen Labor von einem Zahntechniker an einem Modell durchgeführt (ex vivo) werden. Hierzu wird der Zahn nach dem Einsetzen des Wurzelstifts abgeformt und dann ein Positivmodell des zu restaurierenden Zahns angefertigt. Dieses dient als Basis beispielsweise zur Herstellung des Stumpfaufbaus oder der vollständigen Restauration aus Stumpfaufbau und Krone. Die Herstellung von Stumpfaufbau oder Dentalrestauration erfolgt auf die oben beschriebene Weise. Der Stumpfaufbau oder die vollständige Restauration werden dem Patienten anschließend vom Zahnarzt eingesetzt.

## Patentansprüche

1. Formkappe (4) für dentale Stiftaufbauten, welche als fester Bestandteil des Stiftaufbaus geeignet ist, **dadurch gekennzeichnet, dass** sie aus faserverstärktem Kunststoff besteht und einen E-Modul von mindestens 2000 MPa aufweist.

2. Formkappe (4) nach Anspruch 1; **dadurch gekennzeichnet, daß** die Kappenoberfläche polymerisierbare Gruppen und/oder Retentionen aufweist und/oder aufgerauht ist.

3. Formkappe (4) nach einem der Ansprüche 1 bis 2, **dadurch gekennzeichnet, daß** sie durch Polymerisation härtbar ist.

4. Formkappe (4) nach einem der Ansprüche 1 bis 3, **dadurch gekennzeichnet, daß** die Kappe mit einem polymerisierbaren Material (5) gefüllt ist.

5. Formkappe (4) nach Anspruch 3 oder 4, **dadurch gekennzeichnet, daß** die Formkappe (4) und/oder das polymerisierbare Material (5) durch Licht härtbar ist.

6. Formkappe (4) nach Anspruch 4 oder 5, **dadurch gekennzeichnet, daß** die Formkappe (4) bei der Härtung fest mit dem polymerisierbaren.Material (5) verbunden wird.

7. Kit zur Herstellung von Zahn-Stiftaufbauten, enthaltend mindestens einen Verankerungsstift (3), mindestens eine Formkappe (4) gemäß einem der Ansprüche 1 bis 6 und gegebenenfalls polymerisierbares Material (5).

8. Kit nach Anspruch 7, **dadurch gekennzeichnet, daß** er zusätzlich ein Material zur Verblendung der Formkappe (4) enthält.

9. Kit nach Anspruch 7 oder 8, **dadurch gekennzeichnet, daß** der Verankerungsstift (3) aus faserverstärktem Kunststoff besteht.

10. Stiftaufbau enthaltend einen Wurzelstift (3), eine Formkappe (4) gemäß einem der Ansprüche 1 bis 6 und ein Kappenfüllmaterial, **dadurch gekennzeichnet, daß** Wurzelstift (3), Kappenfüllmaterial (5) und Formkappe (4) fest miteinander verbunden sind.

11. Verfahren zur Herstellung von Dentalrestaurationen, **dadurch gekennzeichnet, daß** man eine Formkappe gemäß einem der Ansprüche 1 bis 6 mit polymerisierbarem Material füllt, die gefüllte Formkappe ex vivo auf einen Verankerungsstift aufbringt und dann das polymerisierbare Material härtet, wobei das polymerisierbare Material fest mit der Formkappe verbunden wird.

12. Verfahren nach Anspruch 11, **dadurch gekennzeichnet, daß** man die Formkappe nach dem Härten mit einem Verblendmaterial verblendet.

13. herfahren nach Anspruch 11, **dadurch gekennzeichnet, daß** man eine dentale Krone an der Formkappe befestigt.

14. Verwendung einer Formkappe (4) gemäß einem der Ansprüche 1 bis 6 als festen Bestandteil einer Dentalrestauration.

## Claims

1. Shaping cap (4) for dental pin structures, which is suitable as a permanent constituent of the pin structure, **characterised in that** it consists of a fibre-reinforced plastic and exhibits an E-modulus of at least 2000 MPa.

2. Shaping cap (4) according to Claim 1, **characterised in that** the cap surface exhibits polymerisable groups and/or retentions and/or is roughened.

3. Shaping cap (4) according to one of Claims 1 to 2, **characterised in that** it is curable by polymerisation.

4. Shaping cap (4) according to one of Claims 1 to 3, **characterised in that** the cap is filled with a polymerisable material (5).

5. Shaping cap (4) according to Claim 3 or 4, **characterised in that** the shaping cap (4) and/or the polymerisable material is light curable.

6. Shaping cap (4) according to Claim 4 or 5, **characterised in that** on curing the shaping cap (4) is firmly bonded to the polymerisable material (5).

7. Kit for the preparation of tooth pin structures, comprising at least one anchoring pin (3), at least one shaping cap (4) according to one of Claims 1 to 6 and optionally polymerisable material (5).

8. Kit according to Claim 7, **characterised in that** it additionally comprises a material for veneering the shaping cap (4).

9. Kit according to Claim 7 or 8, **characterised in that** the anchoring pin (3) consists of fibre reinforced plastic.

10. Pin structure comprising a root pin (3), a shaping cap (4) according to one of Claims 1 to 6 and a cap filling material (5), **characterised in that** the root pin (3), the cap filling material (5) and the shaping cap are firmly bonded to each other.

11. Process for the preparation of dental restorations, **characterised in that** a shaping cap according to one of Claims 1 to 6 is filled with polymerisable material, the filled shaping cap is fitted *ex vivo* onto an anchoring pin and then the polymerisable material is cured, wherein the polymerisable material is firmly bonded to the shaping cap.

12. Process according to Claim 11, **characterised in that** after curing, the shaping cap is veneered with a veneering material.

13. Process according to Claim 11, **characterised in that** a dental crown is fixed to the shaping cap.

14. Use of a shaping cap (4) according to one of Claims 1 to 6 as a permanent constituent of a dental restoration.

## Revendications

1. Capuchon profilé (4) pour les superstructures de tenon dentaire qui convient comme partie fixe de la superstructure de tenon, **caractérisé en ce qu'**il consiste en plastique renforcé de fibres et présente un module E d'au moins 2000 MPa.

2. Capuchon profilé (4) selon la revendication 1, **caractérisé en ce que** la surface du capuchon présente des groupes polymérisables et/ou des retenues et/ou qu'elle est rendue rugueuse.

3. Capuchon profilé (4) selon l'une des revendications 1 à 2, **caractérisé en ce qu'**il est durcissable par polymérisation.

4. Capuchon profilé (4) selon l'une des revendications 1 à 3, **caractérisé en ce que** le capuchon est rempli d'une matière polymérisable (5).

5. Capuchon profilé (4) selon la revendication 3 ou 4, **caractérisé en ce que** le capuchon profilé (4) et/ou la matière polymérisable (5) sont durcissables à la lumière.

6. Capuchon profilé (4) selon la revendication 4 ou 5, **caractérisé en ce que** le capuchon profilé (4) s'assemble solidement avec la matière polymérisable (5) lors du durcissement.

7. Kit de fabrication de superstructures de tenon de dent, contenant au moins un tenon d'ancrage (3), au moins un capuchon profilé (4) selon l'une des revendications 1 à 6 et, le cas échéant, de la matière polymérisable (5).

8. Kit selon la revendication 7, **caractérisé en ce qu'**il contient en plus un matériau pour le parement du capuchon profilé (4).

9. Kit selon la revendication 7 ou 8, **caractérisé en ce que** le tenon d'ancrage (3) consiste en plastique renforcé de fibres.

10. Superstructure de tenon, contenant un tenon radiculaire (3), un capuchon profilé (4) selon l'une des revendications 1 à 6 et une matière de remplissage du capuchon, **caractérisée en ce que** le tenon radiculaire (3), la matière de remplissage du capuchon (5) et le capuchon profilé (4) sont solidement assemblés ensemble.

11. Procédé de fabrication de restaurations dentaires, **caractérisé en ce que** l'on remplit un capuchon profilé selon l'une des revendications 1 à 6 par de la matière polymérisable, **en ce que** le capuchon profilé rempli est appliqué *ex vivo* sur un tenon d'ancrage et **en ce qu'**ensuite la matière polymérisable durcit, lors de quoi la matière polymérisable est assemblée solidement avec le capuchon profilé.

12. Procédé selon la revendication 11, **caractérisé en ce que** l'on pare le capuchon profilé avec un matériau de parement après le durcissement.

13. Procédé selon la revendication 11, **caractérisé en ce que** l'on fixe une couronne dentaire au capuchon profilé.

14. Utilisation d'un capuchon profilé (4) selon l'une des revendications 1 à 6 comme composant fixe d'une restauration dentaire.
